(19) Europäisches Patentamt
European Patent Office
Office européen des brevets

(11) Numéro de publication : **0 122 202**
**B1**

# (12) FASCICULE DE BREVET EUROPÉEN

(45) Date de publication du fascicule du brevet : **16.06.87**

(21) Numéro de dépôt : **84400693.2**

(22) Date de dépôt : **09.04.84**

(51) Int. Cl.[4] : **C 07 C 49/497**, C 07 C143/68, C 07 C 45/64, C 07 C 45/68, C 07 C 49/403

(54) **Nouveaux tétra-alkyl-2,2,5,5 cyclohexanone-4 ol-1 et leurs dérivés sulfonylés, leur procédé et leurs intermédiaires de préparation et leur application à la synthèse de lactones cyclopropaniques cis.**

(30) Priorité : **08.04.83 FR 8305772**

(43) Date de publication de la demande : **17.10.84 Bulletin 84/42**

(45) Mention de la délivrance du brevet : **16.06.87 Bulletin 87/25**

(84) Etats contractants désignés : **AT BE CH DE FR GB IT LI LU NL SE**

(56) Documents cités :
**US-A- 3 988 205**
**TETRAHEDRON LETTERS, vol. 24, no. 20, 1983, Pergamon Press Ltd. (GB) J. D'ANGELO et al.: "Synthèse de l'acide cis chrysanthemique", pages 2103-2106.**

(73) Titulaire : **ROUSSEL-UCLAF**
**35, boulevard des Invalides**
**F-75007 Paris (FR)**

(72) Inventeur : **d'Angelo, Jean**
**101, rue du 8 mai 1945**
**F-91300 Massy (FR)**
Inventeur : **Revial, Gilbert**
**69, rue de Bretagne**
**F-75003-Paris (FR)**
Inventeur : **Azerad, Robert**
**13 bis, rue Marcelin Berthelot**
**F-91130 Ris Orangis (FR)**
Inventeur : **Buisson, Didier**
**Les Acacias - 16, rue J. d'Ayen**
**F-28130 Maintenon (FR)**

(74) Mandataire : **Fritel, Hubert et al**
**Département des Brevets ROUSSEL UCLAF B.P. no 9**
**F-93230 Romainville (FR)**

**EP 0 122 202 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet de nouveaux tétra-alkyl-2,2,5,5 cyclohexanone-4 ol-1 et leurs dérivés sulfonylés, leur procédé et leurs intermédiaires de préparation et leur application à la synthèse de lactones cyclopropaniques de structure cis.

La présente invention a ainsi pour objet les tétra-alkyl-2,2,5,5 cyclohexanone-4 ol-1 et leurs dérivés sulfonylés de formule générale (I) :

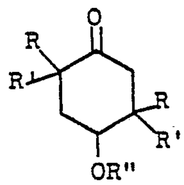

(I)

dans laquelle :

R et R', identiques ou différents, représentent un radical alkyle ayant de 1 à 5 atomes de carbone et R" représente un atome d'hydrogène ou un radical $-SO_2R'''$, dans lequel R''' représente un radical alkyle renfermant de 1 à 5 atomes de carbone ou un radical aryle renfermant de 6 à 14 atomes de carbone.

Dans la formule générale (I), R et R' représentent de préférence un radical méthyle, éthyle, propyle et lorsque R" représente un radical $-SO_2R'''$, R''' représente de préférence un radical méthyle, éthyle, phényle, p-tolyle ou xylyle.

Parmi les composés de formule générale (I), l'invention a notamment pour objet ceux dans lesquels R et R' sont identiques et représentent un radical méthyle.

Parmi les composés de formule générale (I), l'invention a également notamment pour objet ceux dans lesquels R" représente un atome d'hydrogène et ceux dans lesquels R" représente un radical $-SO_2R'''$, dans lesquels R''' représente un radical méthyle ou p-tolyle.

Parmi les composés de formule générale (I), l'invention a plus particulièrement pour objet ceux dans lesquels la configuration de l'atome de carbone porteur du groupement OR" est (S).

L'invention a également pour objet un procédé de préparation des composés de formule (I), telle que définie précédemment, caractérisé en ce que l'on alkyle la cyclohexane dione-1,4 de formule (II) :

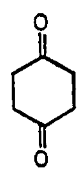

(II)

pour obtenir les tétra-alkyl-2,2,5,5 cyclohexanediones-1,4 de formule (III) :

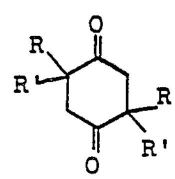

(III)

que l'on réduit de façon sélective pour obtenir les tétraalkyl-2,2,5,5 cyclohexanone-4 ol-1 correspondants, de formule (I), dans laquelle R" représente un atome d'hydrogène, et que l'on transforme, si désiré, lesdits produits en dérivés sulfonylés correspondants, de formule (I) dans laquelle R" représente un radical $-SO_2R'''$, tel que défini précédemment.

Dans des conditions préférentielles d'exécution du stade d'alkylation du procédé de l'invention :

— lorsque les radicaux R et R' sont identiques, la réaction de tétra-alkylation peut être effectuée en une seule étape à partir de la cyclohexane dione-1,4, et d'un halogénure d'alkyle en milieu anhydre, dans le tétrahydrofuranne en présence d'un alcoolate alcalin ;

— lorsque les radicaux R et R' sont différents mais également lorsqu'ils sont identiques, la réaction d'alkylation est effectuée en deux étapes, à partir de cyclohexane dione-1,4 dicarboxylate-2,5 d'éthyle dans les mêmes conditions que celles décrites ci-dessus pour la tétra-alkylation de la cyclohexanedione-1,4.

Ce stade d'alkylation peut être illustré par le schéma réactionnel suivant :

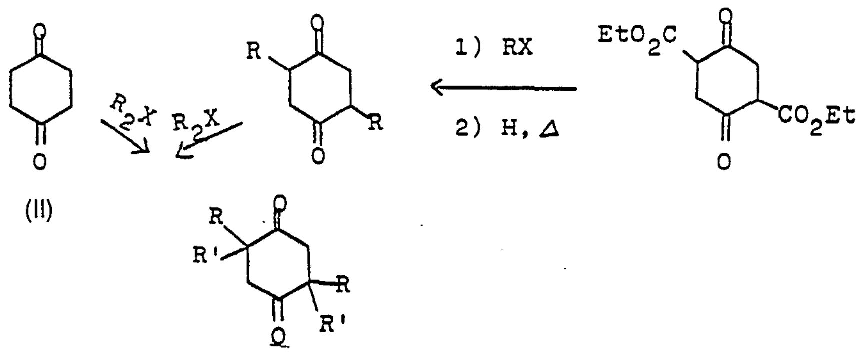


(II)

(III)

$R_2$ pouvant être R ou R',

X = halogène (Cl, Br ou I)

La cyclohexanedione-1,4 (II) de départ est un composé facilement accessible par réduction de l'hydroquinone.

Dans des conditions préférentielles d'exécution du stade de réduction sélective du procédé de l'invention, l'on forme au préalable un monoénolate du composé de formule (III), à l'aide d'un alcoolate alcalin. On opère de préférence en milieu anhydre, dans le tétrahydrofuranne et à une température d'environ 0 °C.

Comme alcoolate alcalin, on peut utiliser un alcoolate de sodium d'un alcool aliphatique inférieur ayant de 1 à 6 atomes de carbone, de préférence le tertiobutylate de sodium ou le tertioamylate de sodium.

La réduction sélective proprement dite est effectuée de préférence à l'aide d'un hydrure tel que l'hydrure de diisobutylaluminium ou d'un borohydrure tel que le borohydrure de sodium.

Dans des conditions très préférentielles d'exécution du stade de réduction sélective du procédé de l'invention, on effectue ladite réduction par voie microbiologique, particulièrement à l'aide d'un fungi imperfecti, notamment choisi dans le groupe constitué par les Curvularia, les Aspergillus, les Mucor, les Geotrichum, les Penicillium, les Rhizopus, les Kloeckera, les Cunninghamella, les Cylindrocarpon, les Fusarium, les Neurospora et les Trichothecium.

L'utilisation des microorganismes ci-dessus présente l'avantage très important de conduire à une réduction sélective et stéréospécifique du composé dicétonique en un cétol de configuration (S).

Parmi les microorganismes ci-dessus, ceux choisis dans le groupe constitué par Curvularia lunata, Aspergillus niger, Aspergillus ochraceus, Mucor racemosus et Penicilium chrysogenum sont tout particulièrement préférés pour le niveau de sélectivité et de stéréospécificité qu'ils permettent d'atteindre.

Le stade de sulfonylation du procédé de l'invention est effectué de préférence à l'aide d'un halogénure d'un acide alkyl ou aryl sulfonique et, plus particulièrement, à l'aide de chlorure de mésyle ou de chlorure de tolyle, en opérant en milieu anhydre, en présence d'une base et à une température d'environ 0 °C.

Les composés de formule (III), telle que définie précédemment, sont des produits nouveaux. A ce titre, les composés de formule (III) et notamment ceux dans lesquels R et R' sont identiques et représentent un radical méthyle, constituent l'un des objets de l'invention.

L'invention a, en outre, pour objet une application des composés de formule (I) telle que définie à la revendication 1, caractérisée en ce que l'on transforme, le cas échéant, les composés de formule (I) dans laquelle R'' représente un atome d'hydrogène, en dérivés sulfonylés, que l'on oxyde à l'aide d'un peracide lesdits dérivés sulfonylés en lactones de formule (IV) :

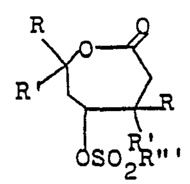

(IV)

puis que l'on effectue en milieu basique une réaction de cyclopropanation desdites lactones (IV) pour obtenir les lactones cyclopropaniques correspondantes de structure cis répondant à la formule générale (V) :

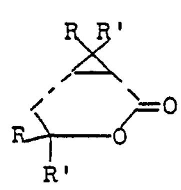

(V)

La réaction d'oxydation du dérivé sulfonylé peut être effectuée à l'aide d'un peracide tel que l'acide perbenzoïque, l'acide monoperphtalique, l'acide peracétique, l'acide métachloroperbenzoïque ou l'acide trifluoroperacétique, mais de préférence en présence d'acide métachloroperbenzoïque, en milieu anhydre, de préférence dans un hydrocarbure halogéné tel que le dichlorométhane, à la température ambiante et pendant un temps compris entre 10 et 100 heures.

La dernière étape de l'application selon l'invention consiste à réaliser la cyclisation intramoléculaire de la lactone sulfonylée (IV) en lactone cis-cyclopropanique de formule (V). Cette cyclisation est réalisée de préférence en présence d'un alcoolate alcalin, en milieu anhydre, de préférence dans du tétrahydrofuranne, et à température d'environ 0 °C.

L'invention a notamment pour objet une application telle que définie précédemment, caractérisée en ce que R et R' sont identiques et représentent un radical méthyle.

L'alcoolate alcalin utilisé plus-haut peut être notamment l'un de ceux qui ont été cités précédemment pour la formation du monoénolate du composé de formule (III).

Les composés de formule (IV), telle que définie précédemment, sont des produits nouveaux. A ce titre, lesdits composés de formule (IV), notamment ceux dans lesquels R et R' sont identiques et représentent un radical méthyle, et tout particulièrement, ceux dans lesquels la configuration de l'atome de carbone porteur du groupement sulfonylé est (S), c'est-à-dire la lactone de l'acide (S) triméthyl-3,3,6 hydroxy-6 mésyloxy-4 heptanoïque, constituent l'un des objets de l'invention.

Les lactones cyclopropaniques de formule (V) constituent des intermédiaires particulièrement précieux dans la synthèse d'acides cis-cyclopropane carboxyliques substitués, notamment dans la synthèse de l'acide cis-chrysanthémique ou acide cis-diméthyl-2,2 (méthyl-2' propényl-1')-3 cyclopropane-1-carboxylique, dont on connait d'une part, les propriétés, notamment insecticides ou olfactives de certains de ses esters et d'autre part, l'intérêt en tant qu'intermédiaires dans la synthèse d'autres esters d'acides cyclopropane carboxyliques dihalogénovinyliques qui possèdent également des propriétés insecticides remarquables.

L'acide cis-chrysanthémique (1R, 3S) constitue ainsi notamment un intermédiaire particulièrement important dans la synthèse de la deltaméthrine découvert par M. ELLIOTT en 1974, ce composé alliant des propriétés insecticides exceptionnelles à une toxicité réduite envers les organismes supérieurs et à une faible persistance dans le milieu naturel.

Cet acide cis-chrysanthémique (1R, 3S) peut donc être obtenu à partir de la lactone de formule (V) de configuration (1R, 3S) dans laquelle R et R' sont identiques et représentent un radical méthyle, elle-même obtenue à partir de la lactone de formule (IV) correspondante de configuration (4S).

La lactone de l'acide cis-diméthyl-2,2 (méthyl-2' propényl-1')-3 cyclopropane-1-carboxylique ou lactone de l'acide cis-chrysanthémique de formule (V) dans laquelle R = R' = $-CH_3$ a déjà été décrite dans le brevet français n° 69.05865 comme intermédiaire dans la préparation de l'acide cis-chrysanthémique racémique, cette lactone étant elle-même obtenue par une suite d'étapes à partir d'acide trans-chrysanthémique.

Les lactones cyclopropaniques de formule (V) constituant des intermédiaires particulièrement précieux dans la synthèse d'acides cyclopropanes carboxyliques substitués en position 3 par une chaîne vinylique ramifiée, la présente demande est particulièrement intéressante d'un point de vue industriel dans la mesure où elle décrit la préparation desdites lactones en peu d'étapes, au départ de produits facilement accessibles et avec des conditions opératoires particulièrement simples et peu coûteuses en énergie.

Par ailleurs, les intermédiaires obtenus se présentent pour la plupart sous forme de produits cristallisés ce qui facilite grandement leur purification lorsque celle-ci s'avère nécessaire.

Les exemples suivants permettent d'illustrer l'invention sans toutefois lui conférer aucun caractère limitatif.

Exemple 1 : Tétraméthyl 2,2,5,5-cyclohexanone-4-ol-1.

Stade A : Tétraméthyl 2,2,5,5-cyclohexanedione-1,4.

A 4,48 g de cyclohexanedione-1,4 et 10,2 ml d'iodure de méthyle, en solution dans 100 ml de tétrahydrofuranne anhydre à 0 °C, on ajoute goutte à goutte 106 ml d'une solution 1,5M de tertio amylate de sodium dans le benzène, en une heure. L'addition terminée, la solution est agitée à 20 °C pendant une heure.

Après hydrolyse par addition d'eau et séparation, la phase aqueuse est extraite à l'éther. Les phases organiques sont combinées puis lavées à l'eau et séchées. Après évaporation, on isole par chromatographie sur colonne de silice (éluant : acétate d'éthyle-hexane 20/80) et cristallisation dans l'hexane, 3,25 g du composé attendu. Rendement : 50 %. Point de fusion : 112 °C.

Spectre IR (Nujol) : 1 700 $cm^{-1}$

Spectre RMN$^1$H (60 MHz, $CCl_4$) : 2,5 (s 4H) 1,1 (s 12H).

Stade A' : Tétraméthyl 2,2,5,5-cyclohexanedione-1,4.

Etape 1 :

a) A 25,6 g de cyclohexanedione-1,4-dicarboxylate-2,5 d'éthyle et 10,4 g d'hydroxyde de sodium en solution dans un mélange de 200 ml d'alcool éthylique à 95° et 50 ml d'eau, on ajoute 13,7 ml d'iodure de méthyle à 0 °C sous agitation. La solution obtenue est ensuite maintenue à 25 °C pendant 100 heures. Après évaporation du solvant, le résidu est repris à l'eau puis extrait à l'éther. La solution éthérée est lavée à l'eau salée et séchée. Après évaporation on obtient 24 g d'huile brute.

b) Les 24 g du produit obtenu ci-dessus sont alors agités dans 200 ml d'acide perchlorique à 20 % à 100 °C pendant 2 heures 30 minutes. Après extraction au chlorure de méthylène et évaporation, le produit brut est chromatographié sur colonne de silice (éluant : acétate d'éthyle-hexane 40/60). On obtient 8,5 g de diméthyl-2,5-cyclohexanedione-1,4 (mélange de 2 diastéréoisomères).

Spectre IR (film) : 1 700 $cm^{-1}$

Spectre RMN$^1$H (60 MHz), $CCl_4$) : 3,0-2,2 (m 6H) 1,1 (dJ = 6Hz 6H).

Etape 2 :

A 3 g de diméthyl-2,5-cyclohexanedione-1,4 et 2,6 ml d'iodure de méthyle en solution dans 30 ml de tétrahydrofuranne anhydre, on ajoute à 0 °C, 28 ml d'une solution 1,5M de tertio amylate de sodium dans du benzène, en 15 minutes. Après hydrolyse à l'eau et séparation, la phase aqueuse est extraite à l'éther. Les phases organiques sont combinées puis lavées à l'eau et séchées. Après évaporation et purification par chromatographie sur colonne de silice (éluant : acétate d'éthyle-hexane 20/80), on obtient 2,9 g de tétraméthyl-2,2,5,5-cyclohexanedione-1,4 (Rendement 82 %) présentant les mêmes caractéristiques que la dione obtenue selon l'exemple 1.

Stade B : Tétraméthyl-2,2,5,5-cyclohexanone-4-ol-1.

A 2 g de tétraméthyl-2,2,5,5-cyclohexanedione-1,4 agitée dans 12 ml de tétrahydrofuranne anhydre, on ajoute goutte à goutte à — 20 °C, 8 ml d'une solution 1,5M de tertio amylate de sodium dans du benzène. La solution obtenue est maintenue une heure à 0 °C puis le mélange est à nouveau porté à — 20 °C et l'on ajoute goutte à goutte 24 ml d'une solution 1M d'hydrure de diisobutylaluminium dans l'hexane. Après 30 minutes à cette température, on ajoute successivement 1 ml d'acétone, 1 ml de méthanol et 10 ml d'acétate d'éthyle jusqu'à précipitation des sels d'aluminium. Après filtration et lavage du précipité à l'éther, la phase organique est évaporée. Le résidu conduit après chromatographie sur colonne de silice (éluant : étherhexane 30/70) à 1,80 g du composé attendu (Rendement : 88 %). Point de fusion (après cristallisation dans l'éther) = 84 °C.

Spectre IR (film) : 3 420 — 1 680 $cm^{-1}$

Spectre RMN$^1$H (60 MHz, $CDCl_3$) : 3,9 (m 1H) 2,6-1,7 (m 5H) 1,2 (s 3H) 1,1 (s 3H) 1,05 (s 3H) 0,9 (s 3H).

Exemple 2 : Mésylate du tétraméthyl-2,2,5,5-cyclohexanone-4-ol-1.

A 600 mg du composé obtenu à l'exemple 1 et 1 ml de triéthyl-λ amine dans 7 ml de chlorure de méthylène anhydre, on ajoute 0,4 ml de chlorure de mésyle à 0 °C sous agitation et l'on maintient 30 minutes à cette température. Après hydrolyse à l'eau et extraction à l'éther, la phase organique est lavée à l'eau salée puis séchée et évaporée. On obtient après chromatographie sur colonne de silice (éluant : acétate d'éthylehexane 40/60) 850 mg de mésylate attendu (Rendement : 97 %). Point de fusion (après cristallisation dans le mélange éther-hexane) = 51 °C.

Spectre IR (nujol) : 1 710 $cm^{-1}$

Spectre RMN$^1$H (60 MHz, $CCl_4$) : 4,8 (dd J = 6 8Hz 1H) 3,0 (s 3H) 2,6-1,9 (m 4H) 1,2 (s 3H) 1,15 (s 3H) 1,10 (s 3H) 0,95 (s 3H).

Exemple 3 : Lactone de l'acide cis diméthyl-2,2-(méthyl-2'-propényl-1')-3-cyclopropane 1-carboxylique.

Stade A : Lactone de l'acide triméthyl-3,3,6-hydroxy-6-mésyloxy-4-heptanoïque.

850 mg de mésylate obtenu selon l'exemple 2 et 1 g d'acide métachloroperbenzoïque sont agités dans 4 ml de chlorure de méthylène anhydre. Après 100 heures, la solution est filtrée, le précipité lavé au chlorure de méthylène et les phases organiques sont combinées puis évaporées. On obtient après chromatographie sur colonne de silice (éluant : acétate d'éthyle-hexane 60/40), 766 mg du produit attendu (Rendement 85 %). Point de fusion (après cristallisation dans le mélange acétate d'éthyle-éther) = 128 °C.

Spectre IR (Nujol) : 1 700 $cm^{-1}$

Spectre RMN$^1$H (60 MHz, $CDCl_3$) : 4,8 (dd J = 6 6Hz 1H) 3,1 (s 3H) 2,75 (m 2H) 2,4 (m 2H) 1,6 (s 6H) 1,2 (s 3H) 1,1 (s 3H).

Stade B : Lactone de l'acide cis-diméthyl-2,2-(méthyl-2'-propényl-1')-3-cyclopropane-1-carboxylique ou lactone de l'acide cis-chrysanthémique.

A 380 mg de la lactone mésylate obtenu selon le stade A dans 4 ml de tétrahydrofuranne anhydre, on ajoute 1,15 ml d'une solution 1,5M de tertio amylate de sodium dans du benzène à 0 °C. On laisse remonter la température à 20 °C en 15 minutes. Après hydrolyse à l'eau, on extrait à l'éther puis lave la phase organique à l'eau salée, sèche et évapore à sec. Après chromatographie sur colonne de silice (éluant : acétate d'éthyle-hexane 40/60), on obtient 220 mg de la lactone cis cyclopropanique attendue (Rendement : 95 %). Fusion = 50,5 °C. Spectre de masse M/e = 168 ($M^+$) 153 124 109 95 81 67 55 43

Spectre IR (film) : 1 720 $cm^{-1}$

Spectre RMN$^1$H (400 MHz, $CDCl_3$) : 1,92 (dd J = 9,7 15,0 Hz 1H) 1,65 (dd J = 5,1 15,0 1H) 1,55 (d J = 7,7 Hz 1H) 1,44 (s 3H) 1,41 (ddd J = 5,1 7,7 9,7 Hz 1H) 1,34 (s 3H) 1,22 (s 3H) 1,08 (s 3H).

Litt. : H. Lehmkuhl, K. Mehler ; Liebigs Ann. Chem 11, 1841 (1978).

Application de la lactone obtenue à l'exemple 3 à la préparation de l'acide cis-chrysanthémique ou cis-diméthyl-2,2-(méthyl-2'-propényl-1')-3-cyclopropane-1-carboxylique racémique.

40 mg de lactone obtenue dans l'exemple ci-dessus sont chauffés en présence de 146 mg de bromure de magnésium hexahydraté dans 0,25 ml de pyridine anhydre à 125 °C pendant 14 heures. Après acidification par 0,5 ml d'acide chlorhydrique 5N, la phase aqueuse est extraite à l'éther ; les phases organiques sont réunies, lavées à l'acide chlorhydrique dilué puis à l'eau salée, puis séchées et évaporées à sec. Après chromatographie sur colonne de silice (éluant : acétate d'éthyle-hexane 20/80), on isole 35 mg d'acide cis-chrysanthémique pur (Rendement : 88 %). L'acide cis-chrysanthémique obtenu est identique à un échantillon authentique décrit par J. Ficini, J. d'Angelo, Tetrahedron Letters (1976) 2441 et J. Ficini, S. Falou, J. d'Angelo, Tetrahedron Letters (1983) 375.

Exemple 4 : (+)-(S)-Tétraméthyl-2,2,5,5-cyclohexanone-4-ol-1.

Curvularia lunata NRRL 2380 conservée sur milieu solide [a] est ensemencée en milieu liquide [b] et cultivée 48 heures à 24 °C dans un incubateur rotatif. La dicétone obtenue selon l'exemple 1 ou 2 est ajoutée en solution à 5 % dans l'éthanol à une concentration finale de 500 mg pour 1 litre de milieu liquide. Après 3 jours d'agitation supplémentaires à 24 °C, la chromatographie en phase vapeur d'un extrait obtenu à l'acétate d'éthyle indique 95 % de conversion en cétol qui est isolé par filtration sur célite, saturation du filtrat par le chlorure de sodium et extraction répétée au dichlorométhane. Après séchage et évaporation, on obtient 460 mg de produit cristallisé jaune pâle qu'on décolore au charbon actif et qu'on recristallise dans l'hexane pour obtenir le (S) (+) cétol (252 mg). Le traitement des eaux mères permet d'obtenir encore 100 mg environ de (S) cétol pur. Rendement : 70 %. Point de fusion (après cristallisation dans l'éther) : 103-104 °C.

Spectre IR (Nujol) : 3 420 — 1 680 $cm^{-1}$

Spectre RMN$^1$H (60 MHz, $CDCl_3$) : 3,9 (m 1H) 2,6-1,7 (m 5H) 1,2 (s 3H) 1,1 (s 3H) 1,05 (s 3H) 0,9 (s 3H).

$/\alpha/_D^{20} = + 89,7°$ (c = 0,3 MeOH).

La chromatographie en phase vapeur d'un dérivé isopropyl uréthane du cétol sur colonne chirale (R α-phényl glycinamide) ne détecte qu'un seul pic, ce qui traduit la présence d'un seul stéréoisomère.

Exemple 5 : (+)-(S)-tétraméthyl-2,2,5,5-cyclohexanone-4-ol-1.

Dans le tableau suivant sont reportés les résultats obtenus en réduisant la dicétone de l'exemple 1 stade A ou A', avec différents microorganismes, en opérant à une concentration de dicétone de 1 g/l.

| Microorganisme | Temps de réaction | % de produit attendu (S) | % de diol formé | % de dicétone résiduaire |
|---|---|---|---|---|
| Curvularia lunata NRRL 2380 | 75 H | 98,2 | - | 1,8 |
| Aspergillus niger | 48 H | 66,2 | 1 | 32,8 |
| Aspergillus ochraceus ATCC 1009 | 46 H | 89,2 | 10,8 | - |
| Mucor racemosus | 75 H | 85,2 | - | 14,8 |

[a]) Milieu solide : Glucose, 20 g, peptone, 5 g, Bacto-Yeast Extract, 5 g, Bacto-Malt Extract, 5 g, Bacto-agar, 20 g, pour 1 litre.

[b]) Milieu liquide (Nakazaki étal., J. Org. Chem., 44 (1979) 4588).

(Suite)

| Microorganisme | Temps de réaction | % de produit attendu (S) | % de diol formé | % de dicétone résiduaire |
|---|---|---|---|---|
| Geotrichum candidum | 119 H | 31,7 | - | 68,3 |
| Penicilium chrysogenum | 119 H | 69,9 | - | 30,1 |
| Rhizopus arrhizus ATCC 11145 | 119 H | 30,5 | - | 69,5 |

De nombreuses souches des espèces Aspergillus niger, Mucor racemosus, Geotrichum candidum et Penicilium chrysogenum sont facilement accessibles dans diverses collections, notamment dans l'ATCC ou le NRRL.

Exemple 6 : Mésylate de (+)-(S) tétraméthyl-2,2,5,5-cyclohexanone-4-ol-1.

A 200 mg du composé de l'exemple 4 et 0,33 ml de triéthylamine dans 3 ml de chlorure de méthylène anhydre, on ajoute 130 ml de chlorure de mésyle à 0 °C sous agitation et l'on maintient 30 minutes à cette température. Après hydrolyse par addition de 2 ml d'eau et extraction à l'éther, la phase organique est lavée par 1 ml d'eau salée puis séchée sur $MgSO_4$ et évaporée. On obtient après chromatographie sur colonne de silice (éluant : acétate d'éthyle-hexane 40/60) 283 mg du mésylate attendu. (Rendement : 97 %).

Point de fusion (après cristallisation dans le mélange étherhexane) = 56-57 °C.

Spectre IR (Nujol) : 1 710 $cm^{-1}$

Spectre RMN$^1$H (60 MHz, $CCl_4$) : 4,8 (dd J = 6,8 Hz 1H) 3,0 (s 3H) 2,6-1,9 (m 4H) 1,2 (s 3H) 1,15 (s 3H) 1,10 (s 3H) 0,95 (s 3H).

$/\alpha/_D^{20} = + 60,7°$ (c = 2,06 $CHCl_3$).

Exemple 7 : Lactone (+) de l'acide cis-diméthyl-2,2-(méthyl-2'-propényl-1')-3-cyclopropane-1-carboxylique.

Stade A : Lactone (+)-(S) de l'acide (S) triméthyl-3,3,6-hydroxy-6-mésyloxy-4-heptanoïque.

283 mg du mésylate obtenu selon l'exemple 6 et 0,33 g d'acide métachloroperbenzoïque sont agités dans 2 ml de chlorure de méthylène anhydre. Après 100 heures, la solution est filtrée, le précipité lavé au chlorure de méthylène et les phases organiques sont combinées puis évaporées. On obtient après chromatographie sur colonne de silice (éluant : acétate d'éthyle-hexane 60/40) 255 mg du produit attendu. (Rendement : 85 %).

Point de fusion (après cristallisation dans le mélange acétate d'éthyle-éther) : décomposition vers 100 °C

Spectre IR (Nujol) : 1 700 $cm^{-1}$

Spectre RMN$^1$H (60 MHz, $CDCl_3$) : 4,8 (dd J = 6 Hz 1H) 3,1 (s 3H) 2,75 (m 2H) 2,4 (m 2H) 1,6 (s 6H) 1,2 (s 3H) 1,1 (s 3H).

$/\alpha/_D^{20} = + 24,7°$ (c = 1,9 $CHCl_3$).

Stade B : Lactone (+) (1R,3S) de l'acide cis-diméthyl-2,2-(méthyl-2'-propényl-1')-3-cyclopropane-1-carboxylique ou lactone (+) de l'acide (1R,3S) cis-chrysanthémique.

A 126 mg de la lactone mésylate obtenue selon le stade A dans 2 ml de tétrahydrofuranne anhydre, on ajoute 0,38 ml d'une solution 1,5 M de tertio amylate de sodium dans le benzène à 0 °C. On laisse remonter la température à 20 °C en 15 minutes. Après hydrolyse à l'eau, on extrait à l'éther puis lave à l'eau salée la phase organique. Après chromatographie sur colonne de silice (éluant : acétate d'éthyle-hexane 40/60), on obtient 73 mg de la lactone cis cyclopropanique attendue (Rendement : 95 %).

Point de fusion (après cristallisation dans l'hexane) 83-84 °C. Spectre de Masse M/e : 168 ($M^+$) 153, 124, 109, 95, 81, 67, 55, 43.

Spectre IR (film) : 1 720 $cm^{-1}$.

Spectre RMN$^1$H (400 MHz, $CDCl_3$) : 1,92 (dd J = 9,7 ; 15,0 Hz 1H) 1,65 (dd J = 5,1 ; 15,0 Hz 1H) 1,55 (d J = 7,7 Hz 1H) 1,44 (s 3H) 1,41 (ddd J = 5,1 ; 7,7 ; 9,7 Hz 1H) 1,34 (s 3H) 1,22 (s 3H) 1,08 (s 3H).

$/ \alpha /_D^{20} = + 78°$ (c = 1,2 dans $CHCl_3$).

(Littérature : S. Torri, T. Inokuchi, R. Oi ; J. Org. Chem. Vol. 48, p. 1944 (1983) : $/ \alpha /_D^{22} = 77,6°$ (c = 1,8

dans $CHCl_3$). F = 83 °C.)

**Revendications**

1. Tétra-alkyl-2,2,5,5 cyclohexanone-4 ol-1 et leurs dérivés sulfonylés de formule générale (I) :

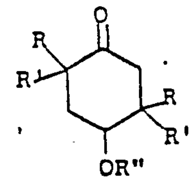

(I)

dans laquelle :

R et R', identiques ou différents, représentent un radical alkyle ayant de 1 à 5 atomes de carbone, et R'' représente un atome d'hydrogène ou un radical $SO_2R'''$, dans lequel R''' représente un radical alkyle renfermant de 1 à 5 atomes de carbone ou un radical aryle renfermant de 6 à 14 atomes de carbone.

2. Tétra-alkyl-2,2,5,5 cyclohexanone-4 ol-1 et leurs dérivés sulfonylés de formule (I) selon la revendication 1, dans laquelle les radicaux R et R' sont identiques et représentent un radical méthyle.

3. Tétra-alkyl-2,2,5,5 cyclohexanone-4 ol-1 de formule (I) selon la revendication 1 ou 2, dans laquelle R'' représente un atome d'hydrogène.

4. Dérivés sulfonylés de tétra-alkyl-2,2,5,5 cyclohexanone-4 ol-1 de formule (I) selon la revendication 1 ou 2, dans laquelle R'' représente un radical $SO_2R'''$, dans lequel R''' représente un radical méthyle ou p-tolyle.

5. Tétra-alkyl-2,2,5,5 cyclohexanone-4 ol-1 de formule (I) selon l'une quelconque des revendications 1 à 4, dans laquelle la configuration de l'atome de carbone porteur du groupement OR'' est (S).

6. Procédé de préparation des composés de formule (I), tels que définis dans l'une quelconque des revendications précédentes, caractérisé en ce que l'on alkyle la cyclohexane dione-1,4 de formule (II) :

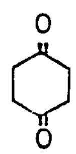

(II)

pour obtenir les tétra-alkyl-2,2,5,5 cyclohexanediones-1,4 de formule (III) :

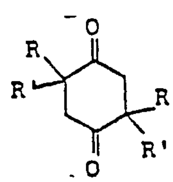

(III)

que l'on réduit de façon sélective pour obtenir les tétraalkyl-2,2,5,5 cyclohexanone-4 ol-1 correspondants de formule (I), dans laquelle R'' représente un atome d'hydrogène, et que l'on transforme, si désiré, lesdits produits en dérivés sulfonylés correspondants, de formule (I) dans laquelle R'' représente un radical $—SO_2—R'''$, tel que défini précédemment.

7. Procédé selon la revendication 6, caractérisé par le fait que l'étape de réduction sélective comprend la formation préalable d'un monoénolate du composé de formule (III).

8. Procédé selon la revendication 7, caractérisé par le fait que l'on forme ledit monoénolate en présence d'un alcoolate alcalin en milieu anhydre et à une température d'environ 0 °C.

9. Procédé selon l'une quelconque des revendications 6 à 8, caractérisé par le fait que la réduction sélective est effectuée à l'aide d'un hydrure.

10. Procédé selon la revendication 9, caractérisé par le fait que ledit hydrure est l'hydrure de diisobutylaluminium ou un borohydrure tel que le borohydrure de sodium.

11. Procédé selon la revendication 6, caractérisé par le fait que l'on effectue la réduction sélective par voie microbiologique.

12. Procédé selon la revendication 11, caractérisé par le fait que l'on effectue la réduction sélective à l'aide d'un fungi imperfecti.

13. Procédé selon la revendication 12, caractérisé par le fait que l'on effectue la réduction sélective à l'aide d'un fungi imperfecti choisi dans le groupe constitué par les Curvularia, les Aspergillus, les Mucor, les Geotrichum, les Penicillium, les Rhizopus, les Kloeckera, les Cunninghamella, les Cylindrocarpon, les

Fusarium, les Neurospora et les Trichothecium.

14. Procédé selon la revendication 11, 12 ou 13, caractérisé par le fait que l'on effectue la réduction à l'aide d'un fungi imperfecti choisi dans le groupe constitué par Curvularia lunata, Aspergillus niger, Aspergillus Ochraceus, Mucor racemosus et Penicillium chrysogenum.

15. Procédé selon l'une quelconque des revendications 6 à 14, caractérisé par le fait qu' à l'étape de sulfonylation, on utilise un halogénure d'un acide alkyl- ou aryl-sulfonique.

16. Procédé selon la revendication 15, caractérisé par le fait que l'halogénure d'acide alkyl- ou aryl-sulfonique est le chlorure de mésyle ou le chlorure de tosyle.

17. A titre de composés industriels nouveaux, les tétra-alkyl-2,2,5,5 cyclohexanediones-1,4 de formule générale (III) :

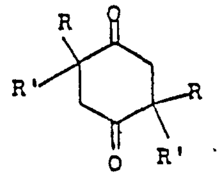
(III)

dans laquelle :

R et R', identiques ou différents, représentent un radical alkyle ayant de 1 à 5 atomes de carbone.

18. Composés selon la revendication 17, caractérisés par le fait que R et R' sont identiques et représentent le radical méthyle.

19. Application des composés selon l'une quelconque des revendications 1 à 5, caractérisée par le fait que l'on transforme, le cas échéant, les composés de formule (I) dans laquelle R" représente un atome d'hydrogène, en dérivés sulfonylés que l'on oxyde à l'aide d'un peracide, lesdits dérivés sulfonylés en lactones de formule (IV) :

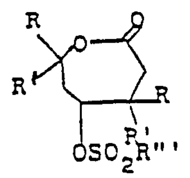
(IV)

puis que l'on effectue en milieu basique une réaction de cyclopropanation desdites lactones (IV) pour obtenir les lactones cyclopropaniques correspondantes de structure cis répondant à la formule générale (V) :

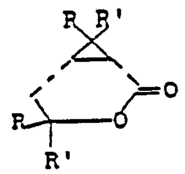
(V)

20. Application selon la revendication 19, caractérisée par le fait que le peracide est l'acide métachloroperbenzoïque.

21. Application selon l'une quelconque des revendications 19 et 20, caractérisée par le fait que la réaction de cyclopropanation est effectuée en présence d'un alcoolate alcalin en milieu anhydre, à température d'environ 0 °C.

22. Application selon l'une quelconque des revendications 19 à 21, caractérisée par le fait que R et R' sont identiques et représentent un radical méthyle.

23. A titre de composés industriels nouveaux, les lactones de formule (IV) :

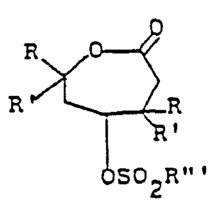
(IV)

dans laquelle R et R', identiques ou différents, représentent un radical alkyle ayant de 1 à 5 atomes de carbone et R‴ représente un radical alkyle ayant de 1 à 5 atomes de carbone ou un radical aryle ayant de 6

à 14 atomes de carbone.

24. Composés selon la revendication 23, caractérisés par le fait que R et R′ sont identiques et représentent un radical méthyle.

25. Composés selon la revendication 23 ou 24, caractérisés par le fait que la configuration du carbone porteur du groupement $OSO_2R'''$ est (S).

**Claims**

1. 2,2,5,5-tetra-alkyl-4-cyclohexanon-1-ol and their sulphonyl derivatives with the general formula (I) :

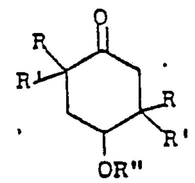

(I)

in which :

R and R′, identical or different, represent an alkyl radical having from 1 to 5 carbon atoms, and R″ represents a hydrogen atom or an $SO_2R'''$ radical in which R‴ represents an alkyl radical containing from 1 to 5 carbon atoms or an aryl radical containing from 6 to 14 carbon atoms

2. 2,2,5,5-tetra-alkyl-4-cyclohexanon-1-ol and their sulphonyl derivatives with the formula (I) according to Claim 1 in which the radicals R and R′ are identical and represent a methyl radical.

3. 2,2,5,5-tetra-alkyl-4-cyclohexanon-1-ol with the formula (I) according to Claim 1 or 2, in which R″ represents a hydrogen atom.

4. Sulphonyl derivatives of 2,2,5,5-tetra-alkyl-4-cyclohexanon-1-ol with the formula (I) according to Claim 1 or 2, in which R″ represents a $SO_2R'''$ radical in which R‴ represents a methyl or p-tolyl radical.

5. 2,2,5,5-tetra-alkyl-4-cyclohexanon-1-ol with the formula (I) according to any one of the Claims 1 to 4, in which the configuration of the carbon carrying the group OR″ is (S).

6. Preparation process for the compounds with the formula (I), as defined in any one of the preceding claims, characterized in that the 1,4-dione cyclohexane with the formula (II) :

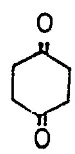

(II)

is alkylized so as to obtain the 2,2,5,5-tetra-alkyl-1,4-cyclohexanedione with the formula (III) :

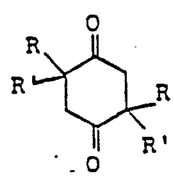

(III)

which is reduced in a selective manner so as to obtain the corresponding 2,2,5,5-tetra-alkyl-4-cyclohexanon-1-ol with the formula (I) in which R″ represents a hydrogen atom and that, if desired, the said products are converted into the corresponding sulphonyl derivatives with the formula (I), in which R″ represents an $—SO_2—R'''$ radical, as previously defined.

7. Process according to Claim 6, characterized by the fact that the stage of selective reduction includes the preliminary formation of a monoenolate of the compound with the formula (III).

8. Process according to Claim 7, characterized by the fact that the said monoenolate is formed in the presence of an alkaline alcoholate in an anhydrous medium and at a temperature of about 0 °C.

9. Process according to any one of the claims 6 to 8, characterized by the fact that the selective reduction is carried out with the help of a hydride.

10. Process according to Claim 9, characterized by the fact that the said hydride is diisobutylaluminium hydride or a borohydride such as sodium borohydride.

11. Process according to Claim 6, characterized by the fact that the selective reduction is carried out by a microbiological route.

12. Process according to Claim 11, characterized by the fact that the selective reduction is carried out with the help of a fungi imperfecti.

13. Process according to Claim 12, characterized by the fact that the selective reduction is carried out with the help of a fungi imperfecti chosen from the group constituted by Curvularia, Aspergillus, Mucor, Geotrichum, Penicillium, Rhizopus, Kloeckera, Cunninghamella, Cylindrocarpon, Fusarium, Neurospora and Trichothecium.

14. Process according to Claim 11, 12, or 13, characterized by the fact that the reduction is carried out with the help of a fungi imperfecti chosen from the group constituted by Curvularia lunata, Aspergillus niger, Aspergillus ochraceus, Mucor racemosus and Penicillium chrysogenum.

15. Process according to any one of the Claims 6 to 14, characterized by the fact that at the stage of sulphonylation, a halogenide of an alkyl- or aryl-sulphonic acid is used.

16. Process according to Claim 15, characterized by the fact that the halogenide of an alkyl- or aryl-sulphonic acid is mesyl chloride or tosyl chloride.

17. As new industrial compounds, the 2,2,5,5-tetra-alkyl-1,4-dionecyclohexanes with the general formula (III) :

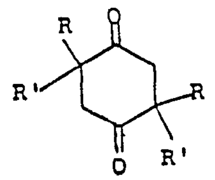

(III)

in which :

R and R', identical or different, represent an alkyl radical having from 1 to 5 carbon atoms.

18. Compounds according to Claim 17, characterized by the fact that R and R' are identical and represent the methyl radical.

19. Use of the compounds according to any one of the Claims 1 to 5, characterized by the fact that, if required, the compounds with the formula (I) in which R" represents a hydrogen atom, are converted into sulphonyl derivatives, that the said sulphonyl derivatives are oxidized by means of a peracid into lactones with the formula (IV) :

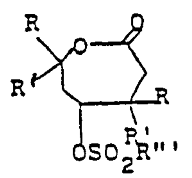

(IV)

then that a cyclopropanation reaction of the said lactones (IV) is carried out in a basic medium so as to obtain the corresponding cyclopropane lactones of cis structure answering to the general formula (V) :

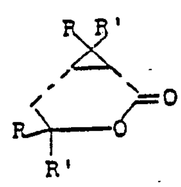

(V)

20. Use according to Claim 19, characterized by the fact that the peracid is metachloroperbenzoic acid.

21. Use according to either one of the Claims 19 and 20, characterized by the fact that the cyclopropanation reaction is carried out in the presence of an alkaline alcoholate in an anhydrous medium, at a temperature of about 0 °C.

22. Use according to any one of the Claims 19 to 21, characterized by the fact that R and R' are identical and represent a methyl radical.

23. As novel industrial compounds, the lactones with the formula (IV) :

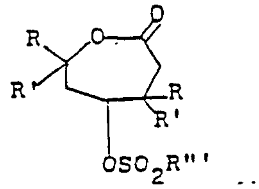

(IV)

in which R and R′, identical or different, represent an alkyl radical having from 1 to 5 carbon atoms and R‴ represents an alkyl radical having from 1 to 5 carbon atoms or an aryl radical having from 6 to 14 carbon atoms.

24. Compounds according to Claim 23, characterized by the fact that R and R′ are identical and represent a methyl radical.

25. Compounds according to Claim 23 or 24, characterized by the fact that the configuration of the carbon carrying the group $OSO_2R'''$ is (S).

**Patentansprüche**

1. 2,2,5,5-Tetraalkyl-cyclohexan-4-on-1-ole und deren Sulfonylderivate der allgemeinen Formel (I)

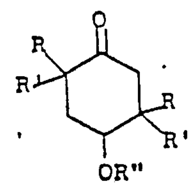

(I)

worin R und R′, die gleich oder voneinander verschieden sind, einen Alkylrest mit 1 bis 5 Kohlenstoffatomen bedeuten und R″ ein Wasserstoffatom oder einen Rest $SO_2R'''$ darstellt, worin R‴ für einen Alkylrest mit 1 bis 5 Kohlenstoffatomen oder einen Arylrest mit 6 bis 14 Kohlenstoffatomen steht.

2. 2,2,5,5-Tetraalkyl-cyclohexan-4-on-1-ole und deren Sulfonylderivate der Formel (I) gemäß Anspruch 1, worin die Reste R und R′ identisch sind und einen Methylrest darstellen.

3. 2,2,5,5-Tetraalkyl-cyclohexan-4-on-1-ole der Formel (I) gemäß Anspruch 1 oder 2, worin R″ ein Wasserstoffatom bedeutet.

4. Sulfonylderivate der 2,2,5,5-Tetraalkyl-cyclohexan-4-on-1-ole der Formel (I) gemäß Anspruch 1 oder 2, worin R″ einen Rest $SO_2R'''$ bedeutet, worin R‴ für einen Methyl- oder p-Tolylrest steht.

5. 2,2,5,5-Tetraalkyl-cyclohexan-4-on-1-ole der Formel (I) gemäß einem der Ansprüche 1 bis 4, worin die Konfiguration des die Gruppe OR″ tragenden Kohlenstoffatoms die (S)-Konfiguration ist.

6. Verfahren zur Herstellung der Verbindungen der Formel (I) gemäß einem der vorhergehenden Ansprüche, dadurch gekennzeichnet, daß man das Cyclohexan-1,4-dion der Formel (II)

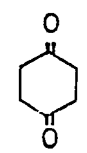

(II)

alkyliert, um die 2,2,5,5-Tetraalkyl-cyclohexan-1,4-dione der Formel (III)

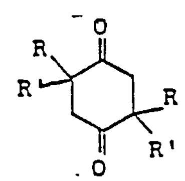

(III)

zu erhalten, die man selektiv reduziert, um die entsprechenden 2,2,5,5-Tetraalkyl-cyclohexan-4-on-1-ole der Formel (I) zu erhalten, worin R″ für ein Wasserstoffatom steht, und gewünschtenfalls diese Produkte in die entsprechenden Sulfonylderivate der Formel (I) überführt, worin R″ für einen Rest $—SO_2—R'''$, wie vorstehend definiert, steht.

7. Verfahren gemäß Anspruch 6, dadurch gekennzeichnet, daß die selektive Reduktionsstufe die vorherige Bildung eines Monoenolats der Verbindung der Formel (III) umfaßt.

8. Verfahren gemäß Anspruch 7, dadurch gekennzeichnet, daß man dieses Monoenolat in Anwesenheit eines Alkalialkoholats in wasserfreiem Milieu und bei einer Temperatur von etwa 0 °C bildet.

9. Verfahren gemäß einem der Ansprüche 6 bis 8, dadurch gekennzeichnet, daß die selektive Reduktion mit Hilfe eines Hydrids durchgeführt wird.

10. Verfahren gemäß Anspruch 9, dadurch gekennzeichnet, daß dieses Hydrid das Diisobutylaluminiumhydrid oder ein Borhydrid, wie Natriumborhydrid, ist.

11. Verfahren gemäß Anspruch 6, dadurch gekennzeichnet, daß man die selektive Reduktion mikrobiologisch durchführt.

12. Verfahren gemäß Anspruch 11, dadurch gekennzeichnet, daß man die selektive Reduktion mit

Hilfe eines Fungi imperfecti durchführt.

13. Verfahren gemäß Anspruch 12, dadurch gekennzeichnet, daß man die selektive Reduktion mit Hilfe eines Fungi imperfecti, ausgewählt unter den Curvularia, den Aspergillus, den Mucor, den Geotrichum, den Penicillium, den Rhizopus, den Kloeckera, den Cunninghamella, den Cylindro carpon, den Fusarium, den Neurospora und den Trichothecium, durchführt.

14. Verfahren gemäß Anspruch 11, 12 oder 13, dadurch gekennzeichnet, daß man die Reduktion mit Hilfe eines Fungi imperfecti, ausgewählt unter Curvularia lunata, Aspergillus niger, Aspergillus ochraceus, Mucor racemosus und Penicillium chrysogenum, durchführt.

15. Verfahren gemäß einem der Ansprüche 6 bis 14, dadurch gekennzeichnet, daß man bei der Sulfonylierungsstufe ein Halogenid einer Alkyl- oder Arylsulfonsäure verwendet.

16. Verfahren gemäß Anspruch 15, dadurch gekennzeichnet, daß das Halogenid der Alkyl- oder Arylsulfonsäure das Mesylchlorid oder Tosylchlorid ist.

17. Als neue, industrielle Produkte die 2,2,5,5-Tetraalkyl-cyclohexan-1,4-dione der allgemeinen Formel (III)

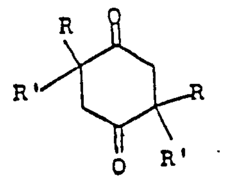

(III)

worin R und R', die identisch oder voneinander verschieden sind, einen Alkylrest mit 1 bis 5 Kohlenstoffatomen darstellen.

18. Verbindungen gemäß Anspruch 17, dadurch gekennzeichnet, daß R und R' identisch sind und den Methylrest bedeuten.

19. Verwendung der Verbindungen gemäß einem der Ansprüche 1 bis 5, dadurch gekennzeichnet, daß man gegebenenfalls die Verbindungen der Formel (I), worin R" ein Wasserstoffatom bedeutet, in Sulfonylderivate überführt, die Sulfonylderivate mit Hilfe einer Persäure zu Lactonen der Formel (IV)

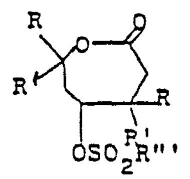

(IV)

oxidiert und anschließend in basischem Milieu eine Cyclopropanierungsreaktion der Lactone (IV) durchführt, um die entsprechenden Cyclopropanlactone mit cis-Struktur der allgemeinen Formel (V)

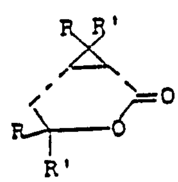

(V)

zu erhalten.

20. Verwendung gemäß Anspruch 19, dadurch gekennzeichnet, daß die Persäure m-Chlorperbenzoesäure ist.

21. Verwendung gemäß einem der Ansprüche 19 und 20, dadurch gekennzeichnet, daß die Cyclopropanierungsreaktion in Anwesenheit eines Alkalialkoholats in wasserfreiem Milieu bei einer Temperatur von etwa 0 °C durchgeführt wird.

22. Verwendung gemäß einem der Ansprüche 19 bis 21, dadurch gekennzeichnet, daß R und R' identisch sind und einen Methylrest bedeuten.

23. Als neue, industrielle Produkte die Lactone der Formel (IV)

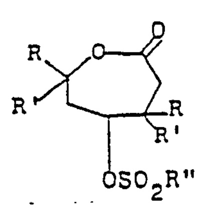

(IV)

worin R und R', die identisch oder voneinander verschieden sind, einen Alkylrest mit 1 bis 5 Kohlenstoffatomen darstellen und R'' einen Alkylrest mit 1 bis 5 Kohlenstoffatomen oder einen Arylrest mit 6 bis 14 Kohlenstoffatomen bedeutet.

24. Verbindungen gemäß Anspruch 23, dadurch gekennzeichnet, daß R und R' identisch sind und einen Methylrest bedeuten.

25. Verbindungen gemäß Anspruch 23 oder 24, dadurch gekennzeichnet, daß die Konfiguration des die Gruppe $OSO_2R'''$ tragenden Kohlenstoffatoms die (S)-Konfiguration ist.